# EUROPÄISCHE PATENTSCHRIFT

(11) **EP 1 737 393 B1**
(45) Veröffentlichungstag und Bekanntmachung des Hinweises auf die Patenterteilung: **09.06.2010**
(21) Anmeldenummer: 04728749.5
(22) Anmeldetag: 22.04.2004
(51) Int. Cl.: A61F 2/16

(54) **VORRICHTUNG ZUM EINSETZEN VERFORMBARER INTRAOCULARLINSEN**
DEVICE FOR INSERTING DEFORMABLE INTRAOCULAR LENSES
DISPOSITIF POUR INTRODUIRE DES CRISTALLINS ARTIFICIELS DEFORMABLES

(43) Veröffentlichungstag der Anmeldung: 03.01.2007
(73) Patentinhaber: Advanced Vision Science, Inc., Goleta, CA 93117 (US)
(72) Erfinder: KAMMERLANDER, Rene, Singapore 189647 (SG); DEINZER, Klaus, CH-8952 Schlieren (CH)
(74) Vertreter: Rehberg Hüppe + Partner
(86) Internationale Anmeldenummer: PCT/CH2004/000242
(87) Internationale Veröffentlichungsnummer: WO 2005/102223

(56) Entgegenhaltungen:
- WO-A-2004/010903
- US-A- 4 681 102
- US-A- 5 354 333
- US-A- 5 578 042
- US-A1- 2003 040 755
- US-A1- 2003 139 749

## Beschreibung

Die Erfindung betrifft eine Vorrichtung zum Einsetzen verformbarer Intraocularlinsen, mit einem Gehäuse, in dem eine Intraocularlinse in einem elastisch verformten Zustand auf nehmbar ist, mit einer Kanüle und mit einem Vorschubmechanismus zum Transportieren der Intraocularlinse durch die Kanüle in ein Auge, wobei der Vorschubmechanismus einen translatorisch bewegbaren Stössel enthält, der dazu bestimmt ist, mit seiner Spitze die Intraocularlinse zu transportieren.

Vorrichtungen zum Einsetzen verformbarer Intraocularlinsen sind bekannt. Ihr Hauptzweck besteht generell darin, den zum Einsetzen einer Intraocularlinse notwendigen Schnitt im Auge möglichst klein halten zu können.

Als Vorschubmechanismen solcher Vorrichtungen sind insbesondere Stössel bekannt, die nach Art einer konventionellen Injektionsspritze für Flüssigkeiten bedient werden, wobei der Stössel translatorisch bewegt wird und mit dieser Bewegung die Intraocularlinse durch die Kanüle in das Auge des Patienten befördert. Als Beispiele für solche Injektoren sei hier auf die Dokumente WO2004/010903A1 oder US6179843B1 verwiesen. Bei der Verwendung derartiger Injektoren wird von vielen Chirurgen als nachteilig empfunden, dass die Bewegung beim Betätigen des Injektors zum Transportieren der Intraocularlinse durch die Kanüle in das Auge in der axialen Richtung des Injektors erfolgt, in der gleichen Richtung, in der zuvor die Kanüle durch einen kleinen Einschnitt in das Auge eingeführt wird. Dadurch wird es schwierig, die Kanüle während der Injektion exakt an ihrer Position im Auge zu halten. Zudem ist es nicht ausgeschlossen, dass die Hand, welche den Stössel betätigt, während der Betätigung abrutscht, was zu einer ruckartigen Axialbewegung der Kanüle und in der Folge zu Verletzungen des Auges führen kann.

Es wurden auch schon Injektoren vorgeschlagen, bei denen eine drehende Betätigung in eine axiale Bewegung des Stössels umgesetzt wird. Ein solcher Injektor ist in der europäischen Patentanmeldung EP1332731A1 (entsprechend US2003/139749) als bekannter Stand der Technik erwähnt. Ein hinten am Injektor angebrachter Betätigungsteil trägt dabei den Stössel und ist mit einem Innengewinde ausgestattet, das mit einem entsprechenden Aussengewinde am Gehäuse zusammenwirkt. Als Nachteil dieses Injektors wird im Dokument erwähnt, dass zu seiner Betätigung zwei Hände benötigt werden. Im gleichen Dokument ist ein verbesserter Injektor beschrieben, der im Prinzip gleich aufgebaut ist, wie der vorangehend beschriebene Injektor mit drehender Betätigung. Um dem Benutzer die Wahl zu lassen, den Injektor mit beiden Händen oder mit nur einer Hand zu bedienen, ist bei diesem verbesserten Injektor die Gewindeverbindung so ausgebildet, dass sie sich dreht, wenn hinten auf den Betätigungsteil ein axialer Druck ausgeübt wird. Durch diese Massnahme kann der verbesserte Injektor wahlweise wie eine Injektionsspritze mit einer Hand oder aber wie der vorangehend beschriebene Injektor durch eine drehende Betätigung bedient werden.

Keiner der vorangehend beschriebenen, bekannten Injektoren trägt dem Umstand Rechnung, dass bei Operationen üblicherweise eine Hilfsperson die Instrumente, insbesondere auch den Injektor, vorbereitet und dann dem Chirurgen reicht, damit dieser die Linse injizieren kann. Zu dieser Vorbereitung gehört im Falle eines Injektors für eine Intraocularlinse das Einsetzen der für den Eingriff benötigten Intraocularlinse in den Injektor und insbesondere das Prüfen, ob sich die Linse in der korrekten, komprimierten, das heisst gefalteten oder aufgerollten Lage befindet und sich durch die Kanüle schieben lässt. Dieser letzte Schritt ist auch bei Injektoren erforderlich, die bereits mit einer eingelegten Intraocularlinse angeliefert werden und die zum einmaligen Gebrauch bestimmt sind. Vorteilhaft wird der zuletzt genannte Vorbereitungsschritt ausgeführt, indem die Intraocularlinse bis in die Kanüle vorgeschoben wird. Wenn die Kanüle aus einem transparenten, das heisst durchscheinenden oder durchsichtigen Material besteht, kann dabei auch optisch überprüft werden, ob die Intraocularlinse richtig gefaltet ist und die für die Injektion gewünschte Lage einnimmt.

Bei den aus dem Stand der Technik bekannten Injektoren ist die vorangehend beschriebene zweistufige Arbeitsweise insofern schwierig, als die Hilfsperson nicht genau weiss, wie weit die Intraocularlinse in die Kanüle vorgeschoben werden darf, bevor der Injektor dem Chirurgen übergeben wird. Sobald nämlich bei der Vorbereitung die Intraocularlinse in der Kanüle zu weit vorgeschoben wird, kann sie nicht mehr zurück bewegt werden, da die Linse, wie erwähnt, mittels eines Stössels in der Kanüle vorgeschoben wird. Wenn die Intraocularlinse bei der Vorbereitung des Injektors so weit vorgeschoben wird, dass ein Teil der Linse das offene Ende der Kanüle überragt, beginnt die Linse, sich durch ihre Eigenelastizität zu entfalten und kann nicht injiziert werden. Unter Umständen wird dadurch nicht nur die betreffende Intraocularlinse unbrauchbar, sondern auch der Injektor, nämlich dann, wenn es sich um einen zum einmaligen Gebrauch bestimmten Injektor handelt.

Ausgehend von diesem Stand der Technik liegt der Erfindung die Aufgabe zugrunde, eine Vorrichtung zum Einsetzen verformbarer Intraocularlinsen derart auszubilden, dass deren Bedienung zwangsläufig in zwei Stufen erfolgt, so dass die Vorrichtung für ein zweistufiges Arbeitsverfahren besonders geeignet ist und die vorangehend genannten Fehler beim Gebrauch der Vorrichtung nicht auftreten können.

Diese Aufgabe wird gemäss der Erfindung dadurch gelöst, dass der Vorschubmechanismus ein erstes, translatorisch betätigbares Betätigungselement und ein zweites, rotatorisch betätigbares Betätigungselement aufweist, dass das erste, translatorisch betätigbare Betätigungselement im Gehäuse durch erste Führungsmittel verdrehsicher geführt ist, dass das zweite, rotatorisch betätigbare Betätigungselement im Gehäuse durch zweite Führungsmittel derart geführt ist, dass es nicht translatorisch beweglich ist, dass das erste, translatorische betätigbare Betätigungselement von einer ersten Position, in der es aus einem Bauteil der Vorrichtung heraus ragt, in eine zweite Position bewegbar ist, in der es nicht aus dem Bauteil heraus ragt, dass der Stössel durch das erste, translatorisch betätigbare Betätigungselement ausgehend von einer Grundposition im Gehäuse nicht weiter als bis in eine Zwischenposition bringbar ist, in der sich die Spitze des Stössels in einem Abstand vom offenen Ende der Kanüle befindet und dass der Stössel durch das zweite, rotatorisch betätigbare Betätigungselement aus der Zwischenposition in eine Endposition bringbar ist, in welcher der Stössel mit seiner Spitze mindestens bis zum offenen Ende der Kanüle reicht.

Diese Ausbildung des Injektors hat insbesondere den Vorteil, dass sich die Arbeitsteilung zwischen Hilfsperson und Chirurg auch auf die Bedienung des Injektors erstreckt und eine Fehlbedienung praktisch ausgeschlossen wird. Dadurch, dass zwei Betätigungselemente vorhanden sind, werden Fehlmanipulationen bei der Vorbereitung der Vorrichtung praktisch ausgeschlossen. Dadurch, dass das erste, translatorische betätigbare Betätigungselement zwei von aussen sichtbare Positionen einnimmt, nämlich eine erste Position, in der es aus einem Bauteil der Vorrichtung heraus ragt und eine zweite Position, in der es nicht aus dem Bauteil heraus ragt, ist jederzeit kontrollierbar, ob die Vorrichtung für die Injektion vorbereitet ist oder nicht.

Wenn nach einer besonderen Ausführungsart der Erfindung die Kanüle transparent oder durchsichtig ist, wird eine optische Kontrolle der Lage der Intraocularlinse in der Kanüle ermöglicht.

Nach einer Ausführungsart der Erfindung ist der Stössel im Gehäuse verdrehsicher geführt. Eine verdrehsichere Führung des Stössels erhöht die Sicherheit, dass die Intraocularlinse in der richtigen Orientierung im Auge platziert werden kann, denn würde der Stössel sich während der Vorschubbewegung drehen, würde durch den Reibungskontakt des Stössels mit der Intraocularlinse in unerwünschter Weise ein Drehmoment auf letztere übertragen.

Mit Vorteil ist das erste Betätigungselement mit dem Stössel unbeweglich und vorzugsweise einstückig verbunden. So kann so die translatorische Betätigungsbewegung sehr einfach und direkt vom ersten Betätigungselement auf den Stössel übertragen werden.

Nach einer weiteren Ausführungsart weisen das erste Betätigungselement und das zweite Betätigungselement Gewindeteile auf, die miteinander in Eingriff kommen, wenn das erste Betätigungselement seine zweite Position erreicht. Somit wird eine rotatorische Betätigung des zweiten Betätigungselements erst wirksam, nachdem das erste Betätigungselement translatorisch betätigt wurde.

Vorteilhaft ist das erste Betätigungselement koaxial im zweiten Betätigungselement aufgenommen und überragt dieses, solange sich das erste Betätigungselement nicht in seiner zweiten Position befindet. Diese Bauweise hat den Vorteil, dass das erste Betätigungselement beispielsweise durch Drücken mit einem Finger, zum Beispiel dem Daumen, nur so weit betätigt werden kann, bis es das zweite Betätigungselement nicht mehr überragt.

Ein Ausführungsbeispiel der Erfindung wird nachfolgend unter Bezugnahme auf die beiliegenden Zeichnungen beispielsweise beschrieben. Es zeigt:
- Fig. 1: eine perspektivische, teilweise geschnittene Ansicht eines Ausführungsbeispiels des Injektors in der Position vor dessen Vorbereitung
- Fig. 2: den Injektor gemäss Figur 1 in der vorbereiteten Position, in der er dem Chirur- gen übergeben wird und
- Fig. 3: den Injektor nach dem Injizieren einer Intraocularlinse.

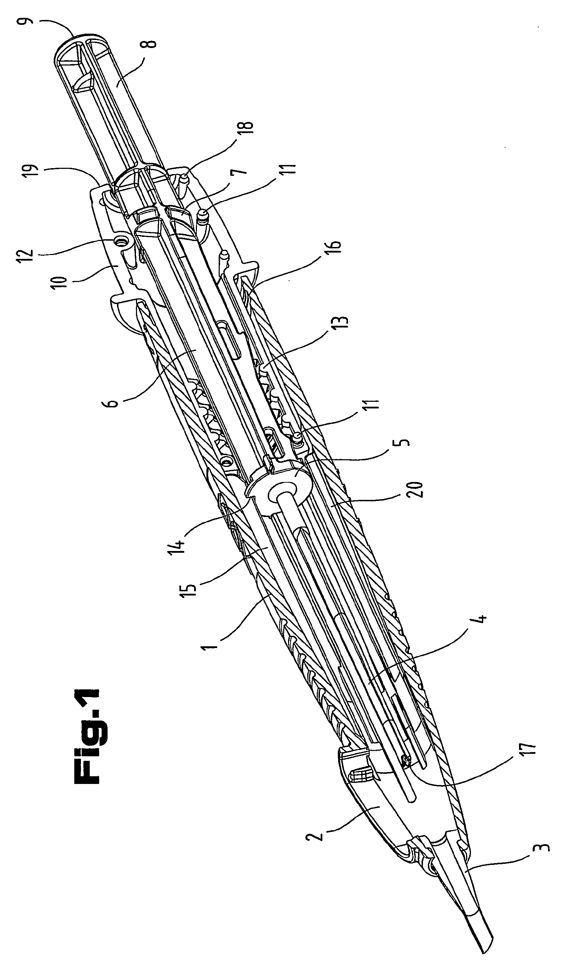

Der in den Figuren 1 bis 3 dargestellte Injektor weist ein Gehäuse 1 auf, in dem die funktionellen Elemente angeordnet sind. Am vorderen Ende des Injektors trägt dieser eine Kanüle 3, durch welche eine komprimierte Intraocularlinse in das Auge eines Patienten injiziert wird. Ein Schacht 2 dient der Aufname einer Linsenalterung, welche in den Figuren nicht dargestellt ist, weil sie für den Gegenstand der vorliegenden Erfindung umwesentlich ist. Im Gehäuse 1 ist ein Stössel 4 gelagert, der die Aufgabe hat, die Intraocularlinse aus der Linsenhalterung zunächst in die Kanüle 3 und später in das Auge eines Patienten zu verschieben, wie dies weiter unten noch genauer beschrieben wird. Der Stössel 4 ist, von vorne nach hinten, mit einem Führungsteil 5, einem Zwischenteil 6 und einem Schiebeteil 8 einstückig verbunden. Am hinteren Ende des Schiebeteils 8 ist ein Betätigungskopf 9 angeformt.

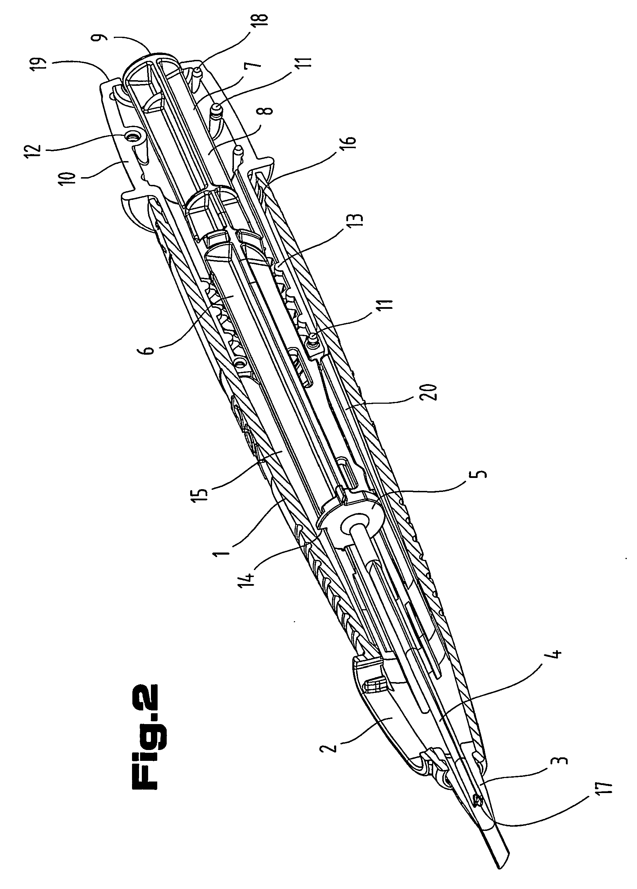

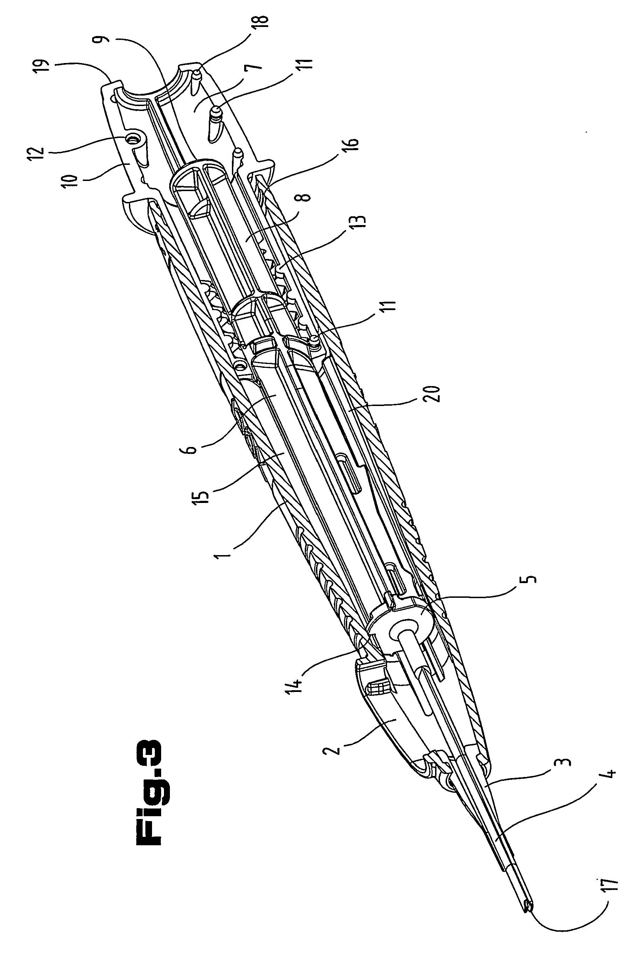

Am hinteren Ende des Gehäuses 1 ist ein Drehteil 10 angeordnet, dar im vorliegenden Beispiel aus zwei schalenartigen Teilen zusammengesetzt ist, die durch Zapfen 11 miteinander verbunden sind, die jeweils in ein entsprechendes Loch 12 am gegenüberliegenden Teil eingreifen. Positionsnocken 18 unterstützen dabei den Zusammenhalt der beiden Teile des Drehteils 10. Das Drehteil 10 ist im Gehäuse rotierbar gehalten, beispielsweise indem er mit einem (nicht gezeigten) Vorsprung in eine am hinteren Ende des Gehäuses 10 angebrachte Ringnut 16 eingreift. Benachbart zu seinem vorderen Ende hat dar Drehteil 10 ein Innengewinde 13, dessen Funktion anlässlich der Beschreibung der Figuren 2 und 3 noch näher erläutert wird.

In der in Figur 1 dargestellten Position wird also eine Hilfsperson eine Intraocularlinse mit Hilfe der Linsenhalterung in verformtem Zustand in den Injektor einsetzen. Danach wird die Hilfsperson den Betätigungskopf 9 bedienen und dadurch den Stössel 4 im Gehäuse 1 nach vorne schieben. Durch diese translatorische Betätigungsbewegung wird die Stösselspitze 17 mit der Intraocularlinse in Kontakt kommen und diese in die Kanüle 3 schieben. Die Bewegung wird durch ein Aussengewinde 7 begrenzt, das am Zwischenteil 6 angeordnet ist und in dem Moment am Innengewinde 13 des Drehteils 10 ansteht, in dem der Betätigungskopf 9 mit der hinteren Stirnfläche 19 des Drehteils 10 bündig ist. Die Komponenten des Injektors sind dabei so dimensioniert, dass in dieser Position die Stösselspitze 17 so weit vor der Mündung der Kanüle 3 steht, dass die Intraocularlinse vollständig in der Kanüle aufgenommen ist. Während der genannten Bewegung gleitet der Führungsteil 5 auf Stützrippen 20, die im Gehäuse 1 angeordnet sind. Zudem greift eine im Gehäuse 1 angeordnete Führungsrippe 15 in einen Schlitz 14 ein, der am Führungsteil 5 angeordnet ist und verhindert so eine Drehung des Stössels 4 und der mit ihm einstückig verbundenen Teile in Bezug auf das Gehäuse 1. Am Ende der translatorischen Betätigungsbewegung nimmt der Injektor die in Figur 2 dargestellte Position ein.

Wie man in Figur 2 sieht, ragt die Stösselspitze 17 nun in die Kanüle 3 hinein und steht in einer solchen Distanz vor der Mündung der Kanüle 3, dass die verformte Intraocularlinse vollständig in der Kanüle 3 aufgenommen ist. Wenn die Kanüle 3 transparent oder durchsichtig ist, kann die Hilfsperson optisch kontrollieren, ob die Intraocularlinse korrekt in der Kanüle 3 sitzt. Ist dies der Fall, wird der Injektor dem Chirurgen zwecks Injektion der Intraocularlinse übergeben.

Der Chirurg übernimmt den Injektor in der in Figur 2 dargestellten Position und führt die Spitze der Kanüle 3 durch einen kleinen Einschnitt in die Linsentasche im Auge des betreffenden Patienten ein. Danach dreht er am Drehteil 10, wobei das Innengewinde 13 des Drehteils 10 mit dem Aussengewinde 7 des Zwischenteils 6 in Eingriff kommt. Da der Stössel 4 durch die Führungsrippe 15 und den Führungsteil 5 gegen Verdrehung im Gehäuse 1 gesichert ist, führt die drehende Betätigung des Drehteils 10 zu einer weiteren Verschiebung des Stössels nach vorne, so dass die Intraocularlinse aus der Kanüle 3 ausgestossen und im Auge platziert wird. Wie man in Figur 3 sieht, ragt am Ende der rotatorischen Betätigungsbewegung die Stösselspitze 17 über die Mündung der Kanüle 3 hinaus, so dass sich die Intraocularlinse sicher in das Auge befördert wird. Dort nimmt die Intraocularlinse in Folge ihrer Elastizität sofort ihre ursprüngliche, unverformte Lage ein.

Das vorangehend beschriebene Ausführungsbeispiel des erfindungsgemässen Injektors ist zum einmaligen Gebrauch bestimmt. Aus der in Figur 3 dargestellten Position kann zwar der Stössel durch Drehung des Drehteils 10 in der entgegengesetzten Richtung wieder zurückgezogen werden, aber nur bis in die Position gemäss Figur 2. Da in dieser Position der Betätigungskopf 9 nicht aus dem Drehteil 10 herausragt und das Gewinde ausser Eingriff gelangt, kann der Stössel 4 nicht weiter nach hinten bewegt und daher auch keine neue Intraocularlinse in den Injektor eingesetzt werden.

### Bezugszeichenaufstellung

- 1: Gehäuse
- 2: Schacht
- 3: Kanüle
- 4: Stössel
- 5: Führungsteil

- 6: Zwischenteil
- 7: Aussengewinde
- 8: Schiebeteil
- 9: Betätigungskopf
- 10: Drehteil

- 11: Zapfen
- 12: Loch
- 13: Innengewinde
- 14: Schlitz
- 15: Führungsrippe

- 16: Ringnut
- 17: Stösselspitze
- 18: Positionsnocken
- 19: Stirnfläche
- 20: Stützrippe

## Patentansprüche

1. Vorrichtung zum Einsetzen verformbarer Intraocularlinsen, mit einem Gehäuse (1), in dem eine Intraocularlinse in einem elastisch verformtem Zustand aufnehmbar ist, mit einer Kanüle (3) und mit einem Vorschubmechanismus (4-10) zum Transportieren der Intraocularlinse durch die Kanüle in ein Auge, **dadurch gekennzeichnet, dass** der Vorschubmechanismus (4-10) so ausgebildet ist, dass durch eine ausschließlich translatorische Betätigungsbewegung am Vorschubmechanismus (4-10) die Intraocularlinse bis in die Kanüle transportierbar ist und dass ausschließlich durch eine rotatorische Betätigungsbewegung am Vorschubmechanismus (4-10) die Intraocularlinse aus der Kanüle ausstoßbar ist.

2. Vorrichtung nach Anspruch 1, **dadurch gekennzeichnet, dass** die Kanüle (3) transparent oder durchsichtig ist.

3. Vorrichtung nach einem der vorhergehenden Ansprüche, **dadurch gekennzeichnet, dass** der Vorschubmechanismus (4-10) einen translatorisch bewegbaren Stößel (4) enthält, der dazu bestimmt ist, mit seinem freien Ende die Intraocularlinse zu transportieren und der vorzugsweise im Gehäuse (1) verdrehsicher geführt ist.

4. Vorrichtung nach einem der vorangehenden Ansprüche, **dadurch gekennzeichnet, dass** der Vorschubmechanismus (4-10) zwei Betätigungselemente enthält, wobei das erste Betätigungselement (9) translatorisch und das zweite Betätigungselement (10) rotatorisch betätigbar ist.

5. Vorrichtung nach einem der Ansprüche 3 bis 4, **dadurch gekennzeichnet, dass** das erste Betätigungselement (9) mit dem Stößel (4) unbeweglich und vorzugsweise einstückig verbunden ist.

6. Vorrichtung nach einem der Ansprüche 3 bis 5, **dadurch gekennzeichnet, dass** das erste Betätigungselement (9) und das zweite Betätigungselement (10) mit Gewindeteilen (13, 18) verbunden sind, die miteinander in Eingriff kommen, wenn der Stößel (4) in die Kanüle (3) ragt.

7. Vorrichtung nach einem der Ansprüche 3 bis 6, **dadurch gekennzeichnet, dass** das erste Betätigungselement (9) koaxial im zweiten Betätigungselement (10) aufgenommen ist und dieses überragt, wenn der Stößel (3) nicht in die Kanüle ragt.

8. Vorrichtung nach einem der Ansprüche 1 bis 2, **dadurch gekennzeichnet, dass** der Vorschubmechanismus (4-10) ein einziges Betätigungselement enthält, welches translatorisch und rotatorisch betätigbar ist, wobei Verriegelungsmittel vorhanden sind, durch welche die rotatorische Betätigungsbewegung erst dann freigegeben wird, wenn die translatorische Bewegung beendet ist.

## Claims

1. Device for inserting deformable intra-ocular lenses, with a housing (1) in which an intra-ocular lense in an elastically deformed condition can be accommodated, with a cannula (3) and with an advancing mechanism (4-10) for transporting the intra-ocular lense through the cannula into an eye, **characterised by** the advancing mechanism (4-10) being designed such that it is possible to transport the intra-ocular lense into the cannula with a pure translational actuating movement at or of the advancing mechanism (4-10) and to eject the intra-ocular lense out of the cannula with a pure rotational actuating movement at or of the advancing mechanism (4-10).

2. Device according to claim 1, **characterised by** the cannula (3) being transparent or translucent.

3. Device according to one of claims 1 and 2, **characterised by** the advancing mechanism (4-10) comprising a plunger (4) which can be moved in the translational direction and intended for transporting at its free end the intra-ocular lense and is in particular guided secured against rotation in the housing (1).

4. Device according to one of claims 1 to 3, **characterised by** the advancing mechanism (4-10) comprising two actuating elements wherein the first actuating element (9) is activated in translational direction and the second actuating element (10) is actuated in rotational direction.

5. Device according to claim 3 or 4, **characterised by** the first actuating element (9) being connected to the plunger (4) in such a manner that it cannot be moved and is preferably connected as one piece with it.

6. Device according to one of claims 3 to 5, **characterised by** the first actuating element (9) and the second actuating element (10) being connected with threaded parts (13, 18), which come into engagement with each other when the plunger (4) extends or protrudes into the cannula (3).

7. Device according to one of claims 3 to 6, **characterised by** the first actuating element (9) being accommodated in coaxial arrangement in the second actuating element (10) and projecting beyond it as long as the plunger (3) does not extend into the cannula.

8. Device according to one of claims 1 and 2, **characterised by** the advancing mechanism (4-10) comprising one single actuating element which is both actuatable in translational and rotational direction, wherein blocking means are provided for freeing the rotational actuating movement at the end of the translational movement.

## Revendications

1. Dispositif pour l'implantation de lentilles intraoculaires déformables, comportant un boîtier (1), dans lequel peut être reçue une lentille intraoculaire à l'état élastiquement déformé, comportant une canule (3) et un mécanisme de poussée (4-10) pour transporter la lentille intraoculaire à travers la canule dans un oeil, **caractérisé en ce que** le mécanisme de poussée (4-10) est configuré de telle sorte que par un mouvement d'actionnement exclusivement en translation du mécanisme de poussée (4-10), la lentille intraoculaire peut être transportée jusque dans la canule, et **en ce que** exclusivement par un mouvement d'actionnement en rotation du mécanisme de poussée (4-10), la lentille intraoculaire peut être poussée hors de la canule.

2. Dispositif selon la revendication 1, **caractérisé en ce que** la canule (3) est transparente ou non opaque.

3. Dispositif selon l'une des revendications précédentes, **caractérisé en ce que** le mécanisme de poussée (4-10) comporte un poussoir (4) mobile en translation, qui est destiné à transporter avec son extrémité libre la lentille intraoculaire et lequel est guidé de manière immobile en rotation de préférence dans le boîtier (1).

4. Dispositif selon l'une quelconque des revendications précédentes, **caractérisé en ce que** le mécanisme de poussée (4-10) contient deux éléments de manoeuvre, le premier élément de manoeuvre (9) pouvant être actionné en translation et le deuxième élément de manoeuvre (10) pouvant être actionné en rotation.

5. Dispositif selon l'une des revendications 3 à 4, **caractérisé en ce que** le premier élément de manoeuvre (9) est relié au poussoir (4) de manière immobile et, de préférence, d'un seul tenant.

6. Dispositif selon l'une des revendications 3 à 5, **caractérisé en ce que** le premier élément de manoeuvre (9) et le deuxième élément de manoeuvre (10) sont reliés par des parties filetées (13, 18) qui engrènent l'une dans l'autre lorsque le poussoir (4) s'avance dans la canule (3).

7. Dispositif selon l'une des revendications 3 à 6, **caractérisé en ce que** le premier élément de manoeuvre (9) est reçu coaxialement dans le deuxième élément de manoeuvre (10) et s'avance au-delà de celui-ci lorsque le poussoir (4) ne s'avance pas dans la canule (3).

8. Dispositif selon l'une des revendications 1 à 2, **caractérisé en ce que** le mécanisme de poussée (4-10) contient un seul élément de manoeuvre, qui peut être actionné en translation et en rotation, des moyens de verrouillage étant prévus, par lesquels le mouvement d'actionnement en rotation est libéré uniquement lorsque le mouvement en translation est terminé.
